# EUROPÄISCHE PATENTANMELDUNG

(11) **EP 0 858 802 A2**
(43) Veröffentlichungstag der Anmeldung: **19.08.1998**
(21) Anmeldenummer: 97122839.0
(22) Anmeldetag: 23.12.1997
(51) Int. Cl.: A61K 9/20, A61K 31/385

(54) **Pharmazeutische Präparate der Thioctsäure zur oralen Anwendung**

(30) Priorität: 13.02.1997 DE 19705555
(71) Anmelder: GLF - Galenik Labor GmbH, 79211 Denzlingen (DE)
(72) Erfinder: Posanski, Ulrich, Dr., 79110 Freiburg i-Br. (DE)
(74) Vertreter: Weisert, Annekäte, Dipl.-Ing. Dr.-Ing.

(57) **Zusammenfassung**

Beschrieben wird eine tablettenförmige Darreichungsform für den Wirkstoff Thioctsäure, die dadurch gekennzeichnet ist, daß
(a) der Wirkstoffgehalt 70 bis 95 Gew.-%, bezogen auf das Gesamtgewicht der Darreichungsform, beträgt,
(b) mindestens 90 Gew.-% des Wirkstoffs eine Partikelgröße kleiner als 200 µm aufweisen und
(c) die Darreichungsform poröses amorphes Siliciumdioxid in einer Menge von 0,2 bis 5 Gew.-%, bezogen auf das Gesamtgewicht der Darreichungsform, und zum Rest pharmazeutisch annehmbare Hilfsstoffe enthält.

## Beschreibung

Die Erfindung betrifft eine tablettenförmige Darreichungsform für den Wirkstoff Thioctsäure (α-Liponsäure) und ein Verfahren zur Herstellung dieser Darreichungsform. α-Liponsäure, 1,2-Dithiolan-3-pentancarbonsäure, (INN: Thioctic acid, siehe *Merck-Index, 12. Ausg. 1996, Nr. 9462*) ist als Leberschutzmittel, z.B. bei Lebererkrankungen, sowie als Neuraltherapeutikum zur Behandlung von Mißempfindungen bei diabetischer Polyneuropathie anwendbar, siehe *Helwig Moderne Arzneimittel 1994, Bd. II, Nr. 43-4.* Bekannte Darreichungsformen sind Injektabilia (intravenös und intramuskulär), sowie Filmtabletten und Kapseln. Gemäss *Helwig, loc. cit.,* beträgt die Dosierung parenteral 300 bis 600 g sowie peroral dreimal täglich 100 bis 200 mg. Als Darreichungsform mit höchster Dosierung sind Filmtabletten mit 600 g Wirkstoff kommerziell erhältlich, siehe Thioctacid ® (Asta Medica), *Rote Liste 1995 Arzneimittelverzeichnis des BPI und VFA, ECV Editio cantor DE-Aulendorf, Nr. 66 008,* welche der bisher anerkannten maximalen Tagesdosis entsprechen (s. Wirkstoffmonographie, veröffentlicht im Bundesanzeiger am 03.11.1990). Zur Erleichterung der Verabreichung und zur Erhöhung der Akzeptanz besteht ein Bedarf an Darreichungsformen mit höherem Wirkstoffgehalt.

Für eine Erhöhung der maximalen Tagesdosis [s. Diabetologia (1995), 38: 1425-1433], z.B. auf 900 mg oder 1200 mg, steht bisher keine geeignete einmal täglich verbreichbare Darreichungsform zur Verfügung. Injektabilia sind ungeeignet, da sie bereits bei den bekannten Dosierungen von 300 bis 600 mg, besonders bei schneller intravenöser Gabe, schwere Störungen bis hin zum Schock, in Einzelfällen auch Krämpfe, Doppeltsehen, Purpura und Thrombopathien verursachen können, siehe *Helwig, loc. cit.* Aufgrund der besseren Verträglichkeit sind daher höher dosierte orale Dar-reichungsformen weniger bedenklich. Die *Rote Liste, loc. cit.,* berichtet nur in vereinzelten Fällen von allergischen Hautreaktionen. Allerdings sind Kapseln als orale Darreichungsformen ungeeignet, da Kapselfüllungen mit einem hohen Wirkstoffgehalt zu voluminös sind. Selbst komprimierte orale Darreichungsformen, wie Tabletten, sind in ihren Abmessungen ebenfalls noch zu groß. Die proportionale Vervielfachung der Bestandteile einer handelsüblichen 200 mg Tablette führt bei der Dosierung von 600 mg pro Tablette bereits zu einem Gesamtgewicht von mehr als 1,2 g pro Tablette.

Tabletten mit einem solch hohen Gewicht sind aufgrund ihrer Größe schlecht einnehmbar und führen wieder zu einer Verschlechterung der Akzeptanz durch den Patienten. Es ist daher erforderlich, den Hilfsstoffanteil in den höher dosierten festen Arzneiformen zu reduzieren. Die Tablettierung von Thioctsäure-Tabletten mit einem hohen Wirkstoffanteil erweist sich als schwierig. Die Preßmassen neigen zur Haftung an den Preßwerkzeugen. Außerdem treten bei den Tabletten kleine Risse parallel zur Oberfläche auf, bei bikonvexen Tabletten kommt es zum Absprengen der Kugelkappen (Deckeln). Diese Störungen des Tablettenaufbaues werden vom Wirkstoff verursacht. Als kritisch erweist sich der niedrige Schmelzpunkt von 60,5°C (R,S-Thioctsäure) bzw. 47°C (R-Thioctsäure) und 46°C (S-Thioctsäure). Zusätzliche Schwierigkeiten treten durch die unbefriedigende chemische Stabilität des Wirkstoffes auf. Die Haltbarkeit der kommerziell erhältlichen Tabletten ist daher begrenzt. Die daraus resultierenden Haltbarkeitsfristen der Handelsprodukte von zwei Jahren verursachen logistische, ökonomische und ökologische Probleme bei der Produktion, dem Absatz und dem Handel.

In der EP 0 676 155 A1 wird eine Arzneimittelformulierung in Form von Tabletten, enthaltend Thioctsäure, mit einem Wirkstoffgehalt von 45 bis 99,9 Gew.-% vorgeschlagen, wobei 20 bis 100% des eingesetzten Wirkstoffes eine Partikelgröße von 100 µm bis 710 µm, vorzugsweise 30 bis 80% eine Partikelgröße von 200 µm bis 710 µm aufweisen. Die mechanischen Eigenschaften dieser Tabletten sind aber unbefriedigend. Insbesondere erweist sich die Auflösungsgeschwindigkeit des Wirkstoffs bei einer derart groben Partikelgrößenverteilung von 100 µm bis 710 µm als nachteilig. Dieser Nachteil liegt unter anderem darin begründet, daß die Auflösungsgeschwindigkeit des Wirkstoffes mit steigender Partikelgröße abnimmt.

Der Erfindung liegt daher die Aufgabe zugrunde, für Thioctsäure eine orale Darreichungsform geeigneter Größe mit hohem Wirkstoffanteil, optimierter Lösungsgeschwindigkeit und verbesserter Stabilität des Wirkstoffes bereitzustellen.

Überraschenderweise wurde gefunden, daß die im Stand der Technik bekannten Tablettenformulierungen durch die Verwendung spezieller Hilfsstoffe in Verbindung mit einer Verringerung der Partikelgröße des Wirkstoffes verbessert werden können.

Gegenstand der Erfindung ist eine tablettenförmige Darreichungsform für den Wirkstoff Thioctsäure, die dadurch gekennzeichnet ist, daß
(a) der Wirkstoffgehalt 70 bis 95 Gew.-%, bezogen auf das Gesamtgewicht der Darreichungsform, beträgt,
(b) mindestens 90 Gew.-% des Wirkstoffes eine Partikelgröße kleiner als 200 µm aufweisen und
(c) die Darreichungsform poröses amorphes Siliciumdioxid in einer Menge von 0,2 bis 5 Gew.-%, bezogen auf das Gesamtgewicht der Darreichungsform, und zum Rest pharmazeutisch annehmbare Hilfsstoffe enthält.

Das erfindungsgemäß verwendete poröse amorphe Siliciumdioxid unterscheidet sich in seinen physikochemischen Eigenschaften grundlegend von dem in der EP 0 667 155 A1 beschriebenen kolloidalen Siliciumdioxid, das dort als sogenanntes "Fließhilfsmittel" verwendet wird. Dieses weist einen geringeren mittleren Partikeldurchmesser im Bereich von 10 bis 40 nm auf, und der pH-Wert einer wäßrigen Dispersion beträgt 3,6 bis 4,3. Es wird durch Hydrolyse von Siliciumtetrachlorid (Flammenhydrolyse) hergestellt und wird als "Aerosil" von der Firma Degussa als Füllstoff für Natur-und Synthetikkautschuke, Verdickungsmittel für die pharmazeutische und kosmetische Industrie, Tablettier- und Dragierhilfsmittel, Antiabsetzmittel und Thixotropiermittel für Anstrichmittel und Druckfarben, usw., vertrieben.

Das erfindungsgemäß verwendete poröse amorphe Siliciumdioxid hingegen wird durch Fällung von Kieselsäure mit Mineralsäuren (sogenannte Fällungskieselsäure) und anschließende Vermahlung des Fällungsprodukts hergestellt. Die poröse amorphe Kieselsäure weist vorzugsweise einen mittleren Partikeldurchmesser von 2000 bis 4000 nm auf und ihre wäßrige Dispersion weist einen pH-Wert von 6,0 bis 8,0 auf.

Das erfindungsgemäß verwendete poröse amorphe Silciumdioxid wird beispielsweise von der Firma W.R. Grace unter der Handelsbezeichnung "Syloid" hergestellt und vertrieben.

Geeignet sind die SYLOID-FP Typen, insbesondere die Typen Al-1-FP, 72-FP, 74-FP und 244-FP, die in der Pharmazie und Kosmetik verwendet werden. Besonders bevorzugt ist poröses amorphes Siliciumdioxid mit einem SiO₂-Gehalt größer als 99% und einer BET-Oberfläche grösser als 20 m²/g. In einer besonders bevorzugten Ausführungsform der Erfindung verwendet man SYLOID 244-FP.

Die tablettenförmige Darreichungsform gemäß vorliegender Erfindung zeichnet sich durch erhöhte Stabilität bei hoher Wirkstoffkonzentration und sehr gutem Auflösungsvermögen des Wirkstoffes aus. Daher läßt sich die Haltbarkeitsfrist auf bis zu 5 Jahre ausdehnen, was im pharmazeutischen Markt für diese Darreichungsformen angestrebt wird.

In einer bevorzugten Ausführungsform der Erfindung beträgt die Wirkstoffmenge 600 bis 900 mg pro Tablette. Die Dimensionen einer tablettenförmigen Darreichungsform mit einer Dosierung von 900 mg verhalten sich wie folgt: 2,5 bis 5,0 (Länge) zu 0,9 bis 2,0 (Breite) zu 1,0 (Höhe). Die Form einer solchen Darreichungsform ist vorzugsweise oblong. Die Grund- und die Deckfläche sind unabhängig voneinander plan oder um die Längsachse konvex gebogen, die Seitenflächen plan, die Stirnflächen beliebig geformt und die Kanten gegebenenfalls abgeschrägt oder abgerundet.

Aufgrund des Vorhandenseins eines chiralen Kohlenstoffatoms in dem 1,2-Dithiolan-Ring der Thioctsäure kann diese in zwei enantiomeren Formen der R- oder S-Form vorliegen. Erfindungsgemäß können beide Isomere entweder im Gemisch, insbesondere als Racemat oder in Enantiomeren reiner Form verwendet werden.

Der Wirkstoffgehalt in der erfindungsgemäßen Darreichungsform beträgt 70 bis 95 Gew.-%, vorzugsweise 75 bis 90 Gew.-%, wobei Dosierungen von 600, 900 und 1200 mg bevorzugt sind.

Mindestens 90 Gew.-% des Wirkstoffes haben eine Partikelgröße kleiner als 200 µm. Gemäß einer bevorzugten Ausführungsform der Erfindung weisen mindestens 50 Gew.-% des Wirkstoffes eine Partikelgröße von kleiner als 100 µm auf.

Die Teilchengröße läßt sich durch konventionelle Mahlverfahren einstellen, wie z.B. durch Mahlung in Kugelmühlen, Stiftmühlen, Mörsermühlen oder Luftstrahlmuhlen. Es wird auf die ausführliche Beschreibung dieser Technik in *Hagers Handbuch der Pharmazeutischen Praxis, 5. vollständig neubearbeitete Auflage, Springer Verlag Berlin, Methoden, Kapitel 3, Seiten 534-549,* verwiesen.

Neben dem porösen amorphen Siliciumdioxid kann die erfindungsgemäße tablettenförmige Darreichungsform für den Wirkstoff Thioctsäure weitere pharmazeutisch annehmbare Hilfsstoffe, die für die Herstellung von festen Darreichungsformen üblich sind, enthalten. So kann die erfindungsgemäße Darreichungsform insbesondere Füll-, Spreng- und Netzmittel, vorzugsweise solche, welche sich für die Direktverpressung eignen, enthalten, wie z.B. Bindemittel vom Typ Zelluloseether, z.B. Methylzellulose, Hydroxypropylmethylzellulose, Ethylzellulose, Hydroxyethylzellulose, Carboxymethylzellulose, Hydroxypropylzellulose, Methylhydroxyethylzellulose, Ethylhydroxyethylzellulose oder Carboxymethylhydroxyethylzellulose.

Die Zelluloseether können trocken in die Pulvermischung eingearbeitet oder in der Granulierflüssigkeit gelöst oder dispergiert eingebracht werden. Bevorzugte Zelluloseethermengen sind 5 bis 20 Gew.-%, insbesondere 8 bis 15 Gew.-%, jeweils bezogen auf die Pulvermischung bzw. getrocknetes Granulat.

Besonders geeignet sind Bindemittel vom Typ Zelluloseether, die auch als Tablettenzerfallsbeschleuniger wirken. Im Handel erhältlich sind niedrigsubstituierte Hydroxypropylzelluloseether von der Fa. Shin-Etsu (L-HPC 20/21/22, u.a.) erhältlich. Diese Zelluloseether haben einen Gehalt an Hydroxypropylgruppen zwischen 5,0 % bis 16,0 %, entsprechend einem molekularen Substitutionsgrad an einer Glucoseeinheit zwischen 0,1 und 0,4, oder Carboxymethylzellulose mit einem Zelluloseethergehalt größer als 95 % (Handelsnamen Tylose® C/CB). Durch die Wahl und den Gehalt an Substituenten sind diese Zelluloseether nur quellbar in Wasser. Die Tablettenzerfallsbeschleuniger können einzeln oder im Gemisch zur Herstellung der erfindungsgemäßen tablettenförmigen Darreichungsform für den Wirkstoff Thioctsäure eingesetzt werden. Das Quellvermögen beeinflußt den Tablettenzerfall in entscheidender Weise (s. Ausführungsbeispiel 5).

Weitere Hilfsstoffe sind z.B. Trockenbindemittel, wie Stärke, z.B. Kartoffel-, Weizen- und Maisstärke, mikrokristalline Zellulose, z.B. Handelsware, welche unter den Warenzeichen Avicel®, Filtrak®, Heweten® oder Pharmacel® kommerziell erhältlich ist, hochdisperses Siliciumdioxid, z.B. Aerosil®, Mannit, Lactose, ferner Polyethylenglykol, insbesondere mit einer Molmasse von 4000 bis 6000, vernetztes Polyvinylpyrrolidon (Polyplasdone®XL oder Kollidon® L), quervernetzte Carboxymethylzellulose (Acdisol®CMC-XL), Natrium- Carboxymethylstärke [Explotab® (Mendell) oder Primojel® (Scholtens)] oder Dicalciumphosphat, z.B. Emcompress®.

Ferner sind Zusätze von geringen Mengen Schmiermittel, z.B. Magnesiumstearat, Natrium - Stearyl-Fumarat, hydrierte Pflanzenöle (Lubritab®, Cutina HR®), Glycerinstearate, -palmitate (Precirol®), Talkum, Stearinsäure oder deren Kombinationen vorteilhaft.

Als Hilfsstoffe sind ferner Fließhilfsmittel, Schmiermittel oder Gegenklebemittel, z.B. siliconisierter Talk, Aluminiumstearat, Calciumstearat, Stearinsäure, Palmitinsäure, Stearinpalmitinsäureester, entfettetes Milchpulver, Stearyl-, Cetyl- und Myristylalkohol, Lanette O, Paraffin oder hydrierte Fette bzw. Öle geeignet.

Die Erfindung betrifft weiterhin ein Verfahren zur Herstellung der erfindungsgemäßen tablettenförmigen Darreichungsform für den Wirkstoff Thioctsäure, das dadurch gekennzeichnet ist, daß man
(a) die in den Ansprüchen 1 bis 8 definierten Komponenten direkt verpreßt oder trocken kompaktiert und das Komprimat einer üblichen Nachbehandlung unterzieht oder
(b) ein Wirkstoffgranulat aus den in den Ansprüchen 1 bis 8 definierten Komponenten mittels Feuchtgranulierung herstellt und dieses Granulat zu einer tablettenförmigen Darreichungsform verpreßt.

### Verfahren a) - Direktverpressung

Das Verpressen zu Tablettenkernen kann mit üblichen Tablettiermaschinen erfolgen. Die Tablettenkerne können unterschiedlich geformt und z.B. rund, oval, oblong, zylindrisch u.a. sein und verschiedene Grössen in Abhängigkeit von der Wirkstoffmenge haben.

In einer bevorzugten Ausführungsform verhalten sich die Dimensionen einer tablettenförmigen Darreichungsform wie folgt: 2,5 bis 5,0 (Länge) zu 0,9 bis 2,0 (Breite) zu 1,0 (Höhe). Die Formgebung einer solchen Darreichungsform ist vorzugsweise oblong. Die Grund- und die Deckfläche sind unabhängig voneinander plan oder um die Längsachse konvex gebogen, die Seitenflächen plan, die Stirnflächen beliebig geformt und die Kanten gegebenenfalls abgeschrägt oder abgerundet.

Die Tabletten können außerdem transparent, farblos, gefärbt und gegebenenfalls beschriftet sein, um diesen Produkten einen zusätzlichen Lichtschutz, ein individuelles Aussehen bzw. sofortige Erkennbarkeit zu verleihen. Die Verwendung von Farbstoffen kann sowohl der Hebung des Aussehens als auch der Kennzeichnung der Präparate dienen. Geeignete zugelassene Farbstoffe bzw. Pigmente sind z.B. Carotinoide, Chlorophyll, Eisenoxide und Titandioxid.

Die durch Direkttablettierung gebildete Preßform kann, falls gewünscht, in an sich bekannter Weise zu einer anderen festen Darrreichungsform weiterverarbeitet werden, z.B. zu Dragées, welche mit einer zusätzlichen Umhüllung, z.B. einer Zucker-, Lack-, Farb- oder Filmschicht, versehen sind. Auf die zahlreichen bekannten Verfahren des Standes der Technik, z.B. Sprühumhüllung in der Wirbelschicht, z.B. nach den bekannten Verfahren mit Geräten der Fabrikate Aeromatic, Glatt, Wurster oder Hüttlin, im Sprühkessel nach dem Verfahren Accela Coata, oder auf das Tauchrohrverfahren, wird verwiesen. Dabei verwendet man die für Sprühumhüllungsverfahren bekannten Hilfsstoffe.

### Verfahren b) - Granulierung:

Granulate sind ebenfalls feste Arzneimittelzubereitungen, welche den Wirkstoff Thioctsäure (α-Liponsäure) und solche Hilfsstoffe enthalten, die in der Pharmazeutischen Technologie für Tablettierverfahren üblich sind, z.B. die weiter vorn für das Verfahren der Direkttablettierung genannten Hilfsstoffe, ferner Netzmittel vom Typ Polyethylenglykole bzw. Ethylenoxidhomopolymere, insbesondere mit einem Polymerisationsgrad von ca. 2,0 x 10³ bis 1,0 x 10⁵ und einem ungefähren Molekulargewicht von ca. 1,0 x 10⁵ bis 5,0 x 10⁶, z.B. unter der Bezeichnung Polyox® (Union Carbide) bekannte Hilfsstoffe, grenzflächenaktive Stoffe, z.B. anionische Tenside vom Typ Alkylsulfat, z.B. Natrium-, Kalium- oder Magnesium-n-dodecylsulfat, -n-tetradecylsulfat, -n-hexadecylsulfat oder -n-octadecylsulfat, Alkylethersulfat, z.B. Natrium-, Kalium- oder Magnesium-n-dodecyloxyethylsulfat, -n-tetradecyloxyethylsulfat, -n-hexadecyloxyethylsulfat oder -n-octadecyloxyethylsulfat oder Alkansulfonat, z.B. Natrium-, Kalium- oder Magnesium-n-dodecansulfonat, -n-tetradecansulfonat, -n-hexadecansulfonat oder -n-octadecansulfonat, nichtionische Tenside vom Typ Fettsäure-Polyhydroxyalkoholester wie Sorbitanmonolaurat, -oleat, -stearat oder -palmitat, Sorbitantristearat oder -trioleat, Polyoxyethylen-Addukte von Fettsäure-Polyhydroxyalkoholestern, wie Polyoxyethylensorbitanmonolaurat, -oleat, -stearat, -palmitat, -tristearat oder -trioleat, Polyethylenglykol-Fettsäureester wie Polyoxyethylstearat, Polyethylenglykol-400-stearat, Polyethylenglykol-2000-stearat, insbesondere Ethylenoxid-Propylenoxid Blockpolymere vom Typ Pluronics® (BWC) oder Synperonic® (ICI).

Als Befeuchtungsmittel können Alkohole mit 2 bis 4 C-Atomen, Ester, z.B. Essigsäureethylester, oder Wasser oder Gemische davon verwendet werden.

Verfahren zur Bildung von Aufbaugranulaten arbeiten kontinuierlich, z.B. durch gleichzeitiges Besprühen der Granuliermasse mit Granulierlösung und Trocknung, z.B. in der Granuliertrommel, in Granulierkesseln, auf Granuliertellern, im Fließbett, durch Sprühtrocknung oder Sprüherstarrung oder diskontinuierlich, z.B. in der Wirbelschicht, im Chargenmischer oder in der Sprühtrocknungstrommel.

Verfahren zur Herstellung von Abbaugranulaten können diskontinuierlich erfolgen, indem die Granuliermasse mit der zugeführten Granulierlösung zunächst ein feuchtes Aggregat bildet, das man anschließend zu Granulaten mit der gewünschten Körnung zerkleinert, wobei man bekannte Extrusions- und Sphäronisationsverfahren anwendet. Als Extruder und Ausrunder sind z.B. Geräte der Firmen Wyss & Probst, Werner & Pfleiderer, HKD, Loser, Fuji, Nica, Caleva, u.a. geeignet.

Die Granuliermasse besteht aus zerkleinertem, vorzugsweise gemahlenem, und dem weiter vorn definierten Wirkstoff und den weiter vorn genannten Hilfsstoffen, z.B. pulverförmigen Füllstoffen, wie mikrokristalliner Zellulose (z.B. diverse AVICEL Typen) - Die Granuliermasse kann je nach angewendetem Verfahren vorgemischt oder durch Zumischen von Wirkstoff zu einem oder mehreren vorgelegten Hilfsstoffen oder durch Zu-mischen der Hilfsstoffe zu vorgelegtem Wirkstoff erhalten werden. Das Granulat wird auf übliche Weise getrocknet, z.B. in der Wirbelschicht bis auf eine Endfeuchte von weniger als 5 %, vorzugsweise weniger als 2 %.

Das Verpressen des Granulates zu Tablettenkernen kann in üblichen Tablettiermaschinen, vorzugsweise Exzenterpressen und Rundläuferpressen, insbesondere Korsch-Exzenter-Tablettiermaschinen oder Rundläuferpressen der Firmen Korsch, Fette, Kilian, Horn, Manesty, Hata., u.a. erfolgen. Besonders geeignet sind Rundläufertablettenpressen mit einer Vordruckeinrichtung.

Zur Maskierung des unangenehmen Geschmacks der Thioctsäure kann die Tablette mit einer Umhüllung versehen werden. Weiterhin verbessert die Umhüllung den Schutz der Thioctsäure vor Sauerstoff und Licht. Die Umhüllung wird mit einem in der pharmazeutischen Industrie bekannten Verfahren aufgetragen.

Die folgenden Beispiele illustrieren die Erfindung ohne sie einzuschränken:

### Beispiele

### Beispiel 1

### Tabletten, enthaltend 600 mg Thioctsäure, mit einem Gesamtgewicht von 700 mg (Direkttablettierung):

Handelsübliche Thioctsäure wird mittels Luftstrahlmahlung (Jet-Mill) zerkleinert. Hierzu wird die Thioctsäure vor dem Mahlprozeß auf ca. 150 Kelvin mit der Hilfe flüssigen Stickstoffs abgekühlt. Der gesamte Mahlvorgang findet unter Stickstoffatmosphäre statt. Vor der Granulation wird die Partikelgrößenverteilung des Wirkstoffes bestimmt. In dem angetrebten Größenbereich (mindestens 90 Gew.-% kleiner als 200 µm) eignet sich die Bestimmung durch Luftstrahlsiebung (z.B. Alpine-Luftstrahlsieb) besonders gut. Falls die angestrebte Partikelgrößenverteilung nicht nach einmaliger Mahlung erreicht wird, muß der Mahlvorgang entsprechend wiederholt werden.

3,0 kg gemahlene Thioctsäure werden durch ein 0,5 mm Sieb gesiebt (Zerstörung von Agglomeraten) und in einen Kubusmischer gefüllt. Zur gesiebten Thioctsäure werden 300 g L-HPC-LH 20 (Firma Shin-Etsu) und 75 g Syloid 244 FP gegeben. Die Pulver werden 5 Minuten miteinander vermischt. Danach werden 125 g gesiebtes Magnesiumstearat zugefügt und die gesamte Pulvermischung nochmals 15 Minuten gemischt. Die Pulvermischung wird anschließend mittels Rundläufertablettenpresse zu Oblongtabletten im Format 18 mm x 8 mm und einem Wölbungsradius von 6 mm verpreßt. Die erhaltenen Tabletten werden anschließend in einem Dragierkessel mittels einer Tauchschwertapparatur mit 1,2 kg einer Filmüberzugsdipersion besprüht.

Die Dispersion hat die folgende Zusammensetzung:

| | |
|---|---|
| Pharmacoat 615 | 4,5 Gew.-% |
| Titandioxid | 1,2 Gew.-% |
| Eisenoxid gelb | 0,3 Gew.-% |
| Polyethylenglykol 4000 | 1,5 Gew.-% |
| destilliertes Wasser | 92,5 Gew.-% |

Das Gesamtgewicht der lackierten und getrockneten Tabletten beträgt ca. 716 mg.

### Beispiel 2

### Tabletten, enthaltend 900 mg Thioctsäure, mit einem Gesamtgewicht von 1140 mg (Wirbelschichtgranulationsverfahren):

5,0 kg gesiebte Thioctsäure werden in eine Wirbelschichtgranulationsapparatur (GPC-G5 (Firma Glatt)/Top Spray-Verfahren) vorgelegt und auf eine Produkttemperatur von 22°C erwärmt. Die Granulation des Wirkstoffes erfolgt mit einer wäßrigen Lösung aus 7 Gew.-% Pharmacoat 615 und 0,2 Gew.-% Na-Laurylsulfat. Insgesamt werden 4,6 kg dieser Lösung zur Granulation der Thioctsäure in das Wirbelbett gesprüht.

Anschließend wird das Granulat auf einen Wassergehalt < 1 Gew.% getrocknet. Das getrocknete Granulat wird durch ein 1,0 mm Sieb egalisiert und in ein Edelstahlfaß gefüllt. Zum Granulat werden 500 g Ac-Di-Sol, 100 g Syloid 244 FP und 200 g gesiebtes Magnesiumstearat gegeben. Das Stahlfaß wird in einen Rhönradmischer gespannt und die Mischung 15 Minuten vermischt. Das fertige Granulat wird auf einer Tablettenpresse zu oblongförmigen Tabletten mit einem Gewicht von 1140 mg verpresst. Eventuell muß die Preßmasse bei Verwendung einer schnellaufenden Rundläuferpresse auf 15°C abgekühlt werden. Die Tabletten können analog zu Beispiel 1 mit einem Überzug versehen werden.

### Beispiel 3

### Tabletten, enthaltend 300 mg Thioctsäure, mit einem Gewicht von 400 mg (Granulationsverfahren mit Intensivmischer):

Vor dem Einfüllen der Thioctsäure in einen Intensivmischer wird die Partikelgröße des Wirkstoffes mikroskopisch (z.B. Zeiss Mikroskop Axiolab) geprüft. Falls die Parkelgröße oberhalb der Spezifikation liegt, muß eine Behandlung des Wirkstoffes durch Mahlung erfolgen (s. Beispiel 1).

1,2 kg gemahlene und gesiebte Thioctsäure werden in einen Lödige-Mischer mit Zerhacker gefüllt. Auf den Wirkstoff werden 120 g in Wasser dispergiertes L-HPC-LH 22 gegeben. Die Wassermenge beträgt ca. 600 g. Die schleimige L-HPC-LH 22 Dispersion wird mittels Zerhacker und den Pflugscharmischwerkzeugen intensiv mit der Thioctsäure vermischt. Falls die erforderliche Konsistenz nicht erreicht wird, kann weiteres destilliertes Wasser zugegeben und untergemischt werden. Die feuchte Mischung wird durch einen Alexanderwerk-Reibschnitzler passiert und anschließend in der Wirbelschicht getrocknet. Zum getrockneten Granulat werden 80 g Syloid 244 FP und 200 g gesiebtes Magnesiumstearat zugefügt und, wie in Beipiel 1 beschrieben, miteinander vermischt. Die Mischung wird zu runden Tabletten mit 10 mm Durchmesser und einem Wölbungsradius von 13 mm verpreßt.

### Beispiel 4

### Tabletten, enthaltend 600 mg Thioctsäure, mit einem Gewicht von 687 mg (Granulation mit Planetenmischer):

1,8 kg gesiebte Thioctsäure und 180 g Klucel ELF werden in einen Erwecka-Plantenmischer mit 900 g destilliertem Wasser durchfeuchtet. Die durchfeuchtete Mischung wird ca. 10 Minuten mittels Plantenmischer durchgeknetet und anschließend durch ein 2,0 mm Sieb passiert (Erwecka-Siebmaschine).

Das feuchte Granulat wird auf Edelstahlblechschalen ausgestrichen und in einem Umlufttrockenschrank auf einen Wassergehalt kleiner 1 Gew.-% getrocknet. Das getrocknete Granulat wird durch ein 1,0 mm Sieb homogenisiert. Dieses Granulat wird in einem Kubusmischer analog zu Beispiel 1 mit 80 g gesiebtem Magnesiumstearat vermischt und zu Oblongtabletten von 687 mg Gewicht verpreßt.

### Beispiel 5

Tabletten mit 600 mg Thioctsäure werden analog zu Beispiel 4 hergestellt. Es wird das Bindemittel Klucel ELF durch Shin-Etsu L-HPC-20 bzw. Pharmacoat 615 ersetzt. Die beiden Bindemittel werden jeweils als trockenes Pulver in den Mengen von 6,7 % , 10,0 % und 13,7 % (m/m) den Pulvermischungen zugefügt und mit destilliertem Wasser durchfeuchtet.

Das pressfertige Granulat wird jeweils zu oblongförmigen Tabletten mit 600 mg Thioctsäure verpresst.

Bei der Zerfallsprüfung der Tabletten konnten die in der Tabelle 1 aufgeführten unterschiedlichen Zerfallsdaten ermittelt werden.

**Tabelle 1**

| Zerfallsprüfung von 600 mg - Thioctsäure nach Arzneibuch | | |
|---|---|---|
| Bindemittel | Konzentration (% - m/m) | Zerfall in dem. Wasser (min) |
| niedrigsubstituierte Hydroxypropylcellulose / NF ,z.B. Shin-Etsu L-HPC ® 20 | 6,7 % | 1 ' 30'' |
| | 10,0 % | 2 ' |
| | 13,7 % | < 1 ' |
| Hydroxypropylmethylcellulose / USP Type 2910 z.B. Pharmacoat ® 615 | 6,7 % | 10 ' |
| | 10,0 % | < 60 ' |
| | 13,7 % | > 60 ' |
| Tablettenformat = 18 x 8 mm / r = 6 mm Tablettendruckfestigkeit 120 k N | | |

## Patentansprüche

1. Tablettenförmige Darreichungsform für den Wirkstoff Thioctsäure, dadurch **gekennzeichnet,** daß
(a) der Wirkstoffgehalt 70 bis 95 Gew.-%, bezogen auf das Gesamtgewicht der Darreichungsform, beträgt,
(b) mindestens 90 Gew.-% des Wirkstoffs eine Partikelgröße kleiner als 200 µm aufweisen und
(c) die Darreichungsform poröses amorphes Siliciumdioxid in einer Menge von 0,2 bis 5 Gew.-%, bezogen auf das Gesamtgewicht der Darreichungsform, und zum Rest pharmazeutisch annehmbare Hilfsstoffe enthält.

2. Tablettenförmige Darreichungsform nach Anspruch 1, dadurch **gekennzeichnet,** daß die Menge des porösen amorphen Siliciumdioxids 0,5 bis 2,5 Gew.-% beträgt.

3. Tablettenförmige Darreichungsform nach Anspruch 1 oder 2, dadurch **gekennzeichnet,** daß der mittlere Partikeldurchmesser des porösen amorphen Siliciumdioxids 2000 bis 4000 nm beträgt.

4. Tablettenförmige Darreichungsform nach den Ansprüchen 1 bis 3, dadurch **gekennzeichnet,** daß das poröse amorphe Siliciumdioxid einen SiO₂-Gehalt von mehr als 99% aufweist und seine BET-Oberfläche größer als 20 m²/g ist.

5. Tablettenförmige Darreichungsform nach den Ansprüchen 1 bis 4, dadurch **gekennzeichnet,** daß der Wirkstoffgehalt 300 mg bis 1200 mg beträgt.

6. Tablettenförmige Darreichungsform nach den Ansprüchen 1 bis 5, dadurch **gekennzeichnet,** daß der Wirkstoff optisch rein in Form seines R-Isomeren oder S-Isomeren, als Racemat oder beliebiges Gemisch dieser Isomeren vorliegt.

7. Tablettenförmige Darreichungsform nach den Ansprüchen 1 bis 6, dadurch **gekennzeichnet,** daß mindestens 50 Gew.-% der Thioctsäure eine Partikelgröße kleiner als 100 µm aufweisen.

8. Tablettenförmige Darreichsformen nach den Ansprüchen 1 bis 7, dadurch **gekennzeichnet,** daß sie ein Bindemittel enthält, das auch als Tablettenzerfallsbeschleuniger wirkt.

9. Tablettenförmige Darreichungsform nach Anspruch 8, dadurch **gekennzeichnet,** daß das Bindemittel ein wasserquellbarer Zelluloseether ist.

10. Tablettenförmige Darreichungsform nach Anspruch 9, dadurch **gekennzeichnet,** daß das Bindemittel eine niedrigsubstituierte Hydroxypropylzellulose ist.

11. Tablettenförmige Darreichungsform nach Anspruch 9, dadurch **gekennzeichnet,** daß das Bindemittel Carboxymethylzellulose mit einem Zelluloseethergehalt größer als 95 % ist.

12. Tablettenförmige Darreichungsform nach Anspruch 9, dadurch **gekennzeichnet,** daß das Bindemittel ein Gemisch aus wasserquellbaren Zelluloseethern, insbesondere aus niedrigsubstituierter Hydroxypropylzellulose und Carboxymethylzellulose mit einem Zelluloseethergehalt größer als 95%, ist.

13. Verfahren zur Herstellung der tablettenförmigen Darreichungsform für den Wirkstoff Thioctsäure nach den Ansprüchen 1 bis 12, dadurch **gekennzeichnet,** daß man
(a) die in den Ansprüchen 1 bis 12 definierten Komponenten direkt verpreßt oder trocken kompaktiert und das Komprimat einer üblichen Nachbehandlung unterzieht oder
(b) ein Wirkstoffgranulat aus den in den Ansprüchen 1 bis 12 definierten Komponenten mittels Feuchtgranulierung herstellt und dieses Granulat zu einer tablettenförmigen Darreichungsform verpreßt.

14. Verwendung von porösem amorphem Siliciumdioxid zur Herstellung von tablettenförmigen Darreichungsformen für den Wirkstoff Thioctsäure.

15. Verwendung von wasserquellbaren Zelluloseethern, die gleichzeitig als Bindemittel und Tablettenzerfallsbeschleuniger wirken, zur Herstellung von tablettenförmigen Darreichungsformen für den Wirkstoff Thioctsäure.
